(19) Europäisches Patentamt
European Patent Office
Office européen des brevets

(11) **EP 4 722 352 A1**

(12) **EUROPEAN PATENT APPLICATION**
published in accordance with Art. 153(4) EPC

(43) Date of publication:
**08.04.2026 Bulletin 2026/15**

(21) Application number: **24815828.9**

(22) Date of filing: **27.05.2024**

(51) International Patent Classification (IPC):
*C12N 7/00* (2006.01)    *A61K 35/76* (2015.01)
*A61P 31/20* (2006.01)    *G01N 33/569* (2006.01)

(52) Cooperative Patent Classification (CPC):
**A61K 35/76; A61P 31/20; C12N 7/00; G01N 33/569**

(86) International application number:
**PCT/KR2024/007169**

(87) International publication number:
**WO 2024/248452 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.05.2023 KR 20230068629**

(71) Applicants:
• **ChoongAng Vaccine Laboratories Co., Ltd
 (CAVAC)
 Daejeon 34055 (KR)**
• **Republic of Korea (Ministry of Environment,
 National Institute of Wildlife Disease Control and
 Prevention)
 Gwangju 62407 (KR)**
• **The Industry & Academic Cooperation in
 Chungnam National University (IAC)
 Daejeon 34134 (KR)**
• **Genkore Inc
 Yuseong-gu, Daejeon 34141 (KR)**
• **AVINEXT CO., LTD.
 Cheongju-si Chungcheongbuk-do 28190 (KR)**

(72) Inventors:
• **YOON, In-joong
 Daejeon 34055 (KR)**
• **LEE, Joo Young
 Daejeon 34055 (KR)**
• **YOO, Sung-sik
 Daejeon 34055 (KR)**
• **KWON, Hyeok-Il
 Daejeon 34055 (KR)**
• **LEE, Seung Chul
 Daejeon 34055 (KR)**
• **KIM, Min Ho
 Daejeon 34055 (KR)**

• **JHEONG, Weonhwa
 Gwangju 62407 (KR)**
• **KIM, Yongkwan
 Gwangju 62407 (KR)**
• **KIM, Yeonji
 Gwangju 62407 (KR)**
• **KIM, Wonjun
 Gwangju 62407 (KR)**
• **KIM, Eunsol
 Incheon 22689 (KR)**
• **LEE, Sanggeon
 Incheon 22689 (KR)**
• **KIM, Sojeong
 Gwangju 62407 (KR)**
• **KIM, Garam
 Gwangju 62407 (KR)**
• **LEE, Song-i
 Gwangju 62407 (KR)**
• **LEE, Seon-Mi
 Incheon 22689 (KR)**
• **PARK, Jae Sung
 Gwangju 62407 (KR)**
• **LEE, Jong-Soo
 Daejeon 34134 (KR)**
• **MO, Inpil
 Cheongju-si Chungcheongbuk-do 28389 (KR)**
• **KIM, Yong-Sam
 Seoul 06591 (KR)**
• **KIM, Do Yon
 Seoul 06591 (KR)**

(74) Representative: **Ipsilon
 12, avenue d'Italie
 75013 Paris (FR)**

Remarks:
The complete document including Reference
Table(s) and the Sequence Listing(s) can be
downloaded from the EPO website

**(Cont. next page)**

EP 4 722 352 A1

(54) **AFRICAN SWINE FEVER VIRUS ASFV-KOR.INJE.MEC-01.2022 ATTENUATED BY CELL ADAPTATION, AND USE THEREOF**

(57) The present invention relates to attenuated African swine fever virus ASFV-KOR.INJE.MEC-01.2022 obtained by passaging the African swine fever virus (ASFV) in a rhesus monkey kidney cell-derived cell line, and uses thereof. The African swine fever virus ASFV-KOR.INJE.MEC-01.2022 naturally attenuated by cell adaptation according to the present invention possesses both safety and efficacy, and therefore it can be effectively utilized as an antigen in vaccine compositions for the prevention of African swine fever.

Figure 4

| % | 0 DPV | 4 DPV | 7 DPV | 11 DPV | 14 DPV | 21 DPV | 28 DPV | 4 DPC | 7 DPC | 10 DPC | 11 DPC | 14 DPC | 18 DPC |
|---|---|---|---|---|---|---|---|---|---|---|---|---|---|
| | 01-05 | 01-09 | 01-12 | 01-16 | 01-19 | 01-26 | 02-02 | 02-06 | 02-09 | 02-12 | 02-13 | 02-16 | 02-20 |
| Group 1 | 100.0 | 100.0 | 100.0 | 100.0 | 100.0 | 100.0 | 100.0 | 100.0 | 100.0 | 100.0 | 100.0 | 100.0 | 100.0 |
| Group 2 | 100.0 | 100.0 | 100.0 | 100.0 | 100.0 | 100.0 | 100.0 | 100.0 | 100.0 | 66.7 | 0.0 | 0.0 | 0.0 |

**Description**

TECHNICAL FIELD

The present invention relates to African swine fever virus ASFV-KOR.INJE.MEC-01.2022 attenuated by cell adaptation and uses thereof.

This work was supported by Science and Engineering Research Infrastructure Development Project of National Research Foundation of Korea (Project Number: 2021R1A6A1A03045495).

BACKGROUND ART

African swine fever (ASF) is a viral disease that primarily infects domestic pigs and wild boars. After the initial infection, symptoms typically appear within 2 to 10 days. Common symptoms include loss of appetite, weakness, reddened skin, inflammation of the conjunctiva, vomiting, bloody diarrhea, and fever. Additionally, the skin may turn blue, necrotic patches (i.e., black spots) may develop, and hemorrhaging may occur. In pregnant sows, ASF can lead to spontaneous abortion. The symptoms of ASF are similar to those of classical swine fever, and depending on the virulence of the virus, the mortality rate among infected animals ranges from 30% to 100%.

The African swine fever virus (ASFV) is a large double-stranded DNA virus belonging to the family *Asfarviridae.* Its DNA genome varies significantly in length, ranging from 160 to 210 kbp depending on the isolate. The genome contains 150 to 167 open reading frames (ORFs) that encode 54 structural proteins of the ASFV particle and more than 100 infection-related proteins (Dixon et al., 2013. Virus Res 173: 3-14). To date, 24 different genotypes of ASFV have been identified.

As of now, there are no globally developed vaccines or treatments for ASF. ASFV is currently classified into 24 different genotypes, and the greater the variety of virus strains, the more challenging vaccine development becomes. With the recent global rise in African swine fever cases, countries like the United States and China are accelerating vaccine development, but commercialization is expected to take time. Currently, African swine fever has been reported in 53 countries worldwide, including 15 European countries, 29 African countries, and 9 Asian countries. In Asia, where African swine fever is geographically close to South Korea, the disease was first detected in China in August 2018, followed by outbreaks in Mongolia, Vietnam, Cambodia, Laos, Myanmar, the Philippines, and North Korea.

Meanwhile, Korean Patent Publication No. 2021-0065128 discloses an "African swine fever virus vaccine" that relates to a recombinant nucleic acid molecule containing an expression cassette encoding a polyepitope composed of T-cell antigens derived from African swine fever virus proteins. Additionally, Korean Patent Publication No. 2021-0127887 describes polypeptides, polynucleotides, and plasmids involved in generating an immune response against African swine fever and an "African swine fever vaccine composition" containing them. However, there is no disclosure regarding the "African swine fever virus ASFV-KOR.INJE.MEC-01.2022 attenuated by cell adaptation and used thereof" described in the present invention.

DETAILED DESCRIPTION OF INVENTION

TECHNICAL PROBLEMS TO BE SOLVED

The present invention is devised in view of the need described above. Specifically, during the process of developing a susceptible cell line for an isolate (wild type) of the African swine fever virus, the inventors of the present invention found that the African swine fever virus, which was being passaged after infecting a cell line derived from rhesus monkey kidney cells, is transformed into an attenuated form, and subsequently found that the naturally attenuated African swine fever virus is safe and effective as a vaccine strain, thereby completing the present invention.

TECHNICAL MEANS FOR SOLVING THE PROBLEMS

To solve the problems that are described in the above, the present invention provides attenuated African swine fever virus ASFV-KOR.INJE.MEC-01.2022 obtained by passaging the African swine fever virus (ASFV) in a rhesus monkey kidney cell-derived cell line.

The present invention further provides a vaccine composition for prevention of African swine fever, comprising the attenuated ASFV-KOR.INJE.MEC-01.2022 as an effective component.

The present invention further provides a method for preventing African swine fever by administering the vaccine composition to an animal of the family *Suidae.*

The present invention further provides a kit for detecting African swine fever virus, which comprises the attenuated ASFV-KOR.INJE.MEC-01.2022 as an effective component.

The present invention further provides a method for diagnosing African swine fever infection comprising reacting the attenuated ASFV-KOR.INJE.MEC-01.2022 with antiserum isolated from a suspected African swine fever-infected individual.

The present invention still further provides a method for producing a vaccine against African swine fever comprising passaging the attenuated ASFV-KOR.INJE.MEC-01.2022 in a vaccine-producing cell line.

ADVANTAGEOUS EFFECT OF INVENTION

The African swine fever virus ASFV-KOR.INJE.MEC-01.2022 naturally attenuated by cell adaptation according to the present invention possesses both safety and efficacy. Therefore, it can be effectively utilized as an antigen in vaccine compositions for prevention of African swine fever.

BRIEF DESCRIPTION OF THE DRAWINGS

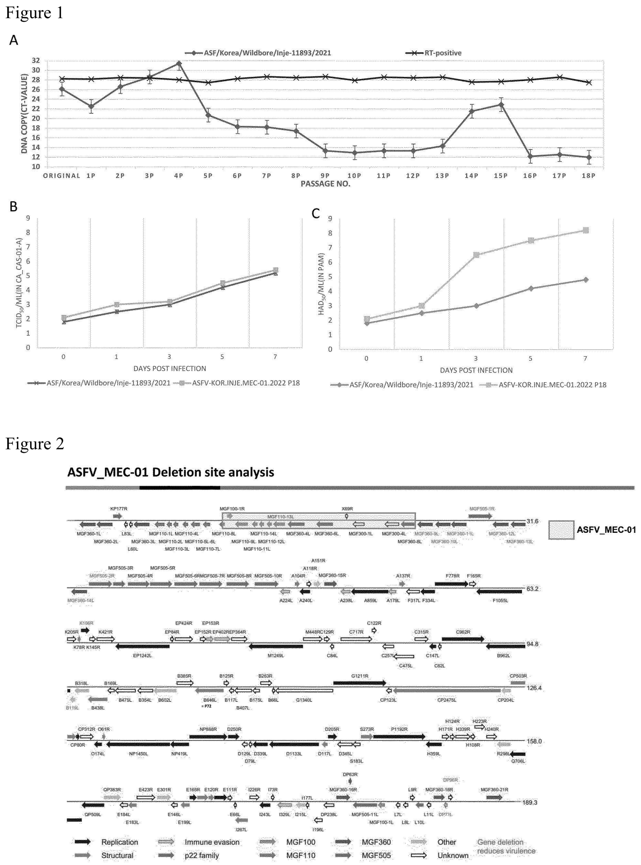

Figure 1 illustrates the followings: Figure 1A shows the results of determining the qPCR Ct value using the ASFV P72 gene to figure out the cell adaptation process of the ASFV isolate according to 18 passages after infecting and passaging a domestic wild boar ASFV isolate (ASF/Korea/Wildbore/Inje-11893/2021) in the CA-CAS-01-A cell line, and Figures 1B and 1C show the results of measuring the virus titer of a single vaccine candidate (ASFV-KOR.INJE.MEC-01.2022) of passage 18, which has been finally obtained through plaque assay in CA-CAS-01-A cell line, in which B shows the result of determining $TCID_{50}$ (tissue culture infectious dose) in CA-CAS-01-A cell line and C shows the result of determining $HAD_{50}$ (median hemadsorption unit) in primary porcine macrophages.

Figure 2 illustrates a schematic diagram showing the genomic deletion sites of ASFV-KOR.INJE.MEC-01.2022 (ASFV_MEC-01), produced in CA-CAS-01-A cells infected with the domestic wild boar ASFV isolate (ASF/Korea/-Wildbore/Inje-11893/2021).

Figure 3 illustrates a schematic diagram of the animal test conducted to evaluate the safety and efficacy of the vaccine candidate strain, ASFV-KOR.INJE.MEC-01.2022.

Figure 4 shows the survival rate of test animals after two doses of the vaccine candidate (ASFV-KOR.INJE.MEC-01.2022) administered at a two-week interval, followed by challenge infection with the ASFV_Hwacheon strain (i.e., domestic wild boar ASFV isolate) and observation for three weeks.

Figure 5 shows the body temperature changes in test animals following vaccination with the vaccine candidate (ASFV-KOR.INJE.MEC-01.2022) and subsequent challenge infection. The normal temperature range is between 38°C and 39.5°C.

Figure 6 shows the result of measuring the antibody titers in animal test using multi-antigen indirect ELISA analysis for p32, p62, and p72 proteins of ASFV. S/P (sample-to-positive) values are interpreted as follows: ≤30%: Negative, >30% to <40%: Suspected, and ≥40%: Positive

BEST EMBODIMENT(S) FOR CARRYING OUT INVENTION

To achieve the object that is described in the above, the present invention provides attenuated African swine fever virus ASFV-KOR.INJE.MEC-01.2022 (hereinbelow, ASFV_MEC-01) obtained by passaging the African swine fever virus (ASFV) in a rhesus monkey kidney cell-derived cell line.

The attenuated African swine fever virus ASFV_MEC-01 of the present invention is characterized by the deletion of genes that are associated with attenuation. Preferably, one or more genes selected from the group consisting of *MGF 100-1R, ASFV G ACD 00190, MGF 110-9L, MGF 110-10L, MGF 110-14L, ASFV G ACD 00240, MGF 110-12L, MGF 110-13La, MGF 110-13Lb, ASFV G ACD 00270, MGF 360-4L, MGF 360-6L, MGF 360-8L partial, MGF 300-1L, MGF 300-2R, MGF 300-4L, MGF 110-8L partial, ASFV G ACD 00210, ASFV G ACD 00300, ASFV G ACD 00320, ASFV G ACD 00330, ASFV G ACD 00350, ASFV G ACD 00360* and *X69R* are deleted. More preferably, all of the following genes may be deleted: *MGF 100-1R, ASFV G A CD 00190, MGF 110-9L, MGF 110-10L, MGF 110-14L, ASFV G ACD 00240, MGF 110-12L, MGF 110-13La, MGF 110-13Lb, ASFV G ACD 00270, MGF 360-4L, MGF 360-6L, MGF 360-8L partial, MGF 300-1L, MGF 300-2R, MGF 300-4L, MGF 110-8L partial, ASFV G ACD 00210, ASFV G ACD 00300, ASFV G ACD 00320, ASFV G ACD 00330, ASFV G ACD 00350, ASFV G ACD 00360* and *X69R,* but the present invention is not limited thereto.

Furthermore, the attenuated African swine fever virus, ASFV_MEC-01 of the present invention may additionally comprise, based on the ASFV Georgia 2007/1 genome sequence (GenBank: FR682468.2), the following substitutions: the 2,329th base adenine (A) is changed to guanine (G), the 7,059th base cytosine (C) is changed to thymine (T); the 26,425th and 27,200th base thymine (T) is changed to cytosine (C), the 44,576th base adenine (A) is changed to guanine (G); the 72,295th, 72,315th, and 72,316th base guanine (G) are changed to adenine (A), and cytosine (C), which is the

179,097th, 180,319th, 180,607th, 180,619th, 180,718th, 181,034th, 181,322nd, 181,809th, 181,891st and 182,053rd base, is changed to thymine (T) (see Table 6); and the above base mutations may be the following: the 106th amino acid leucine (Leu, L) of the MGF 360-1La protein is changed to proline (Pro, P), the 197th amino acid tryptophan (Trp, W) of the MGF 110-1L protein is changed to a stop codon, the 69th and 329th amino acids asparagine (Asn, N) of the MGF360-10L protein is changed to serine (Ser, S), the 323rd amino acid lysine (Lys, K) of the MGF 505-9R protein is changed to glutamic acid (Glu, E), and the 14th and 422nd amino acids methionine (Met, M) and alanine (Ala, A) of the MGF505-11L protein are changed to isoleucine (Ile, I) and threonine (Thr, T), respectively; the 96th and 100th amino acids aspartic acid (Asp, D) and glutamic acid (E) of the MGF 100-1L protein are changed to asparagine (N) and lysine (K), respectively, and the 86th amino acid alanine (A) of the MGF 100-3L protein is changed to threonine (T), but the present invention is not limited thereto.

In one embodiment of the present invention, the genome sequence of the attenuated African swine fever virus ASFV_MEC-01 may be composed of the nucleotide sequence of SEQ ID NO: 3.

The attenuated ASFV_MEC-01 of the present invention is characterized by having a viral titer of at least $1 \times 10^8$ $HAD_{50}$/mL in primary porcine macrophages.

Additionally, in the present invention, the rhesus monkey kidney cell-derived cell line may be the CA-CAS-01-A cell line, which has the deposit number KCTC 14568BP.

The present invention further provides a vaccine composition for prevention of African swine fever, comprising the attenuated ASFV_MEC-01 as an effective component.

In the vaccine composition of the present invention, the attenuated African swine fever virus ASFV_MEC-01 is as described above and has a genome consisting of the nucleotide sequence of SEQ ID NO: 3.

Form of the vaccine composition according to the present invention may be selected from the group consisting of a live vaccine, an inactivated vaccine, a subunit vaccine produced using a gene of an attenuated African swine fever virus, a vector vaccine, a chimeric vaccine, a DNA and RNA vaccine, but is not limited thereto.

In the present invention, the term "prevention" means any act of suppressing or delaying the occurrence, spread, and recurrence of African swine fever infection by administering the vaccine composition of the present invention.

In addition, the vaccine composition of the present invention may additionally include a pharmaceutically acceptable carrier and/or adjuvant. In addition to the carrier, the vaccine composition of the present invention may additionally include an excipient and/or a diluent.

The term "pharmaceutically acceptable" as used herein refers to a substance that is physiologically tolerable and does not typically cause allergic reactions such as gastrointestinal upset, dizziness or the like when administered to a mammal. Examples of such carriers, excipients and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. Additionally, it may contain fillers, anticoagulants, lubricants, wetting agents, fragrances, emulsifiers, and preservatives.

In addition, the "adjuvant" as used herein means a pharmaceutical or immunological agent administered for the purpose of enhancing the immune response of a vaccine. As the adjuvant, aluminum hydroxide, aluminum phosphate, alum (potassium aluminum sulfate), MF59, virosome, AS04 [a mixture of aluminum hydroxide and monophosphoryl lipid A (MPL)], AS03 (a mixture of DL-$\alpha$-tocopherol, squalene, and polysorbate 80, an emulsifier), CpG, Flagellin, Poly I:C, AS01, AS02, ISCOMs, or ISCOMMATRIX can be used.

The vaccine composition of the present invention can be formulated using methods known in the pertinent art to enable rapid release, or sustained or delayed release, of the effective component when administered to a mammal. The formulations include powders, granules, tablets, emulsions, syrups, aerosols, soft or hard gelatin capsules, sterile injectable solutions, and sterile powders.

The vaccine composition according to the present invention can be administered by various routes, for example, orally, parenterally, for example, suppository, transdermal, intravenous, intraperitoneal, intramuscular, intralesional, nasal, intrathecal administration, and also can be administered using an implantable device for sustained release or continuous or repeated release. The number of administrations can be once a day or divided into several times within a desired range, and the administration period is not particularly limited.

The present invention further provides a method for preventing African swine fever by administering the vaccine composition to an animal of the family *Suidae.*

In the method for preventing African swine fever according to the present invention, the animal of the family *Suidae* may preferably be a wild boar (genus *Sus)* animal to which a domesticated species (*Sus scrofa*) belongs, but is not limited thereto.

The present invention further provides a kit for detecting African swine fever virus, which comprises the attenuated ASFV_MEC-01 as an effective component.

The kit may include a reagent capable of detecting the attenuated African swine fever virus ASFV_MEC-01 according to the present invention and an antibody specifically binding to the attenuated African swine fever virus ASFV_MEC-01. In the kit of the present invention, the attenuated African swine fever virus ASFV_MEC-01 functions as an antigen, and the reagent for detecting the virus (antigen)-antibody complex may include, but is not limited to, a reagent for radial immunoassay, ELISA (Enzyme linked immunosorbent assay), or immunofluorescence analysis.

The present invention further provides a method for diagnosing African swine fever infection comprising reacting the attenuated ASFV_MEC-01 with antiserum isolated from a suspected African swine fever-infected individual.

The term "individual" used in the present invention means a mammal that has developed or can develop African swine fever infection, and may preferably include a pig or a wild boar.

The method for diagnosing African swine fever infection according to the present invention may consist of a step of reacting a sample (i.e., antiserum) isolated from a suspected African swine fever individual with the African swine fever virus ASFV_MEC-01 of the present invention under conditions in which an antigen/antibody complex can be formed, and then detecting the formation of the antigen/antibody complex. The method for detecting the formation of the antigen/antibody complex can be performed through a method that is well known in the pertinent art.

The present invention still further provides a method for producing a vaccine against African swine fever comprising passaging the attenuated ASFV_MEC-01 in a vaccine-producing cell line.

In the production method of the present invention, the vaccine-producing cell line may preferably be the cell line CA-CAS-01-A deposited with the deposit number KCTC14568BP, but is not limited thereto, and there is no limitation on the type of cell as long as it is a cell in which infection and proliferation of the attenuated African swine fever virus ASFV_MEC-01 of the present invention can occur.

In the production method of the present invention, the attenuated African swine fever virus ASFV_MEC-01 or the antigens derived therefrom can be converted into a physiologically acceptable form. This process can be carried out based on experience in the production of a vaccine against influenza. For example, to prepare a vaccine injection, virus particles can be lyophilized (i.e., freeze-dried) in 100 mL of phosphatebuffered saline (PBS) in the presence of 1% human albumin and 2% peptone in an ampoule, preferably in a glass ampoule. Alternatively, the vaccine injection may be produced by sequential freeze-drying of the virus in the preparation. The preparation may contain additional additives such as mannitol, dextran, sugars, glycine, lactose or polyvinylpyrrolidone or other auxiliaries such as antioxidants or inert gases, stabilizers or recombinant proteins suitable for *in vivo* administration (e.g. human serum albumin). The glass ampoules are then sealed and can be stored for several months between 4°C and room temperature. However, unless otherwise required, the ampoules can preferably be stored at below -20°C.

Hereinbelow, the present invention is explained in greater detail in view of Examples. However, the following Examples are given only for exemplification of the present invention and it is evident that the scope of the present invention is not limited by them.

EXAMPLES

Materials and Methods

1. Cell and Virus

Monkey kidney cell-derived CA-CAS-01-A cell line (deposit number: KCTC14568BP) was maintained in $\alpha$-MEM (Invitrogen) medium containing 5% fetal bovine serum (FBS; Invitrogen) and antibacterial and antifungal solution (100x; Invitrogen) at 37°C in a humidified 5% $CO_2$ incubator under passaging every 2 to 3 days. As for the African swine fever virus (ASFV), a domestic isolate (ASF/Korea/Wildbore/Inje-11893/2021) isolated from a wild boar, which had occurred in Inje, Gangwon-do, South Korea in 2022, and provided by the Korean National Institute of Wildlife Disease Management, was used.

2. Virus passaging

ASFV domestic isolate (ASF/Korea/Wildbore/Inje-11893/2021) was adapted to the CA-CAS-01-A cell line, and qPCR (quantitative PCR) was performed using a primer set specific for the ASFV P72 gene (Table 1). Then, ASFV culture with a Ct value of 22 or less (Ct value: approximately 18) was used in the experiment.

A serum-free medium was used as a medium for ASFV infection, and 3 to 4 days after the infection, it was replaced with the virus growth medium (i.e., serum-free medium) used for infection, and the previously sensitized virus was removed to determine the propagated virus. Passaging was performed at 7-day intervals after the infection, and the culture broth of the 1st, 3rd, 5th, and 10th passages were collected, the supernatants and cells were frozen at -80°C, and then thawed once in a 37°C constant temperature water bath. qPCR was then performed using the culture broth to measure the Ct value, and a test to determine intracytoplasmic infection (Alkaline Phosphatase assay_ASFV P30 detection; 1st antisera: Rabbit anti African swine Fever virus phosphoprotein p30 antisera, 2nd antibody: Goat Anti-rabbit IgG H&L, Alkaline phosphate substrate kit: ImmPACT Vector RED) was carried out. When it is found to be ASFV-positive, 2 mL of the first-passage culture broth ($1 \times 10^6$ CFU/mL) was used along with 8 mL of serum-free medium for the next passage. This process was repeated for subsequent passages.

[Table 1]

| Information of primers used for qPCR | | |
| --- | --- | --- |
| Primer Name | Nucleotide Sequence (5'→3') | SEQ ID NO: |
| P72 Forward | CTG CTC ATG GTA TCA ATC TTA TCG A | 1 |
| P72 Reverse | GAT ACC ACA AGA TC(AG) GCC GT | 2 |

**3. Virus titer measurement**

From CA-CAS-01-A cell line infected with the ASFV isolate, culture supernatant was collected at the time point when 70% CPE (cytopathic effect) occurred after the infection. For growth kinetics experiment, the supernatant was collected at different time points from cells infected with each virus strain, and then stored at -80°C. Virus titer was examined by quantitative real-time RT-PCR using the primer sets described in above Table 1. Quantitative real-time RT-PCR employed the following conditions: 1 cycle of 50°C for 2 minutes and 95°C for 10 minutes for the pretreatment process, and 40 cycles of 95°C for 15 seconds and 60°C for 1 minute for the main process.

**4. Nucleotide sequencing**

The whole-length nucleotide sequence of the cell-adapted ASFV was analyzed by Celemics, Inc. upon request.

**5. Determination of antibody titer (ELISA)**

ASF virus P32, P62, and P72 were detected by ELISA and antibody titers were measured. The measurement was performed using the ID ScreenA® African Swine Fever Indirect (ASF Elisa) kit (IDvet). Briefly, blood collected from the vaccinated group and negative group of the test animals was centrifuged at 4,500 rpm for 10 minutes, and the supernatant (serum) was collected. 190 µl of dilution buffer were added to each well, 10 µl of the negative control group were added to wells A1 and B1, and 10 µl of the positive control group were added to wells C1 and D1. The serum samples collected above were added to each of the remaining wells, and then reacted at 21°C (+5) for 45 minutes (±4 minutes). After that, the reactants in each well were removed, washed three times with 300 µl of wash buffer, and 100 µl of concentrated conjugate which has been diluted 1/10 using dilution buffer were added to each well and reacted at 21°C (+5) for 30 minutes (±3 minutes). After removing the reactants in each well, washing was carried out three times with 300 µl of wash buffer, 100 µl of substrate solution were added to each well, and the reaction was allowed to occur for 15 minutes (+2 minutes) in the dark at 21°C (+5). Thereafter, 100 µl of reaction stop solution were added and the absorbance was measured at 450 nm.

The validity of the reaction results is determined based on the following criteria:

- Average OD value of the positive control is greater than 0.350 (ODPC > 0.350).
- Ratio of the average OD value of the positive control to the average OD value of the negative control is greater than 3 (ODPC/ODNC > 3).
- Sample-to-positive percentage (S/P%) for each sample is calculated using the following formula:

$$S/P\,\% = \{(OD_{sample} - ODNC) / (ODPC - ODNC)\} \times 100,$$

in which interpretation of S/P% values is made as follows:

$$S/P\% \leq 30\% \rightarrow \text{Negative}$$

$$30\% < S/P\% < 40\% \rightarrow \text{Suspicious}$$

$$S/P\% \geq 40\% \rightarrow \text{Positive.}$$

**6. Pig infection test**

In the present invention, *in vivo* pig studies were conducted in accordance with the guidelines of the Institutional Animal Care and Use Committee (IACUC), and the tests were carried out at a biosafety level 3 (BSL-3) research facility within the Wildlife Disease Control Center of the Ministry of Environment, Korea.

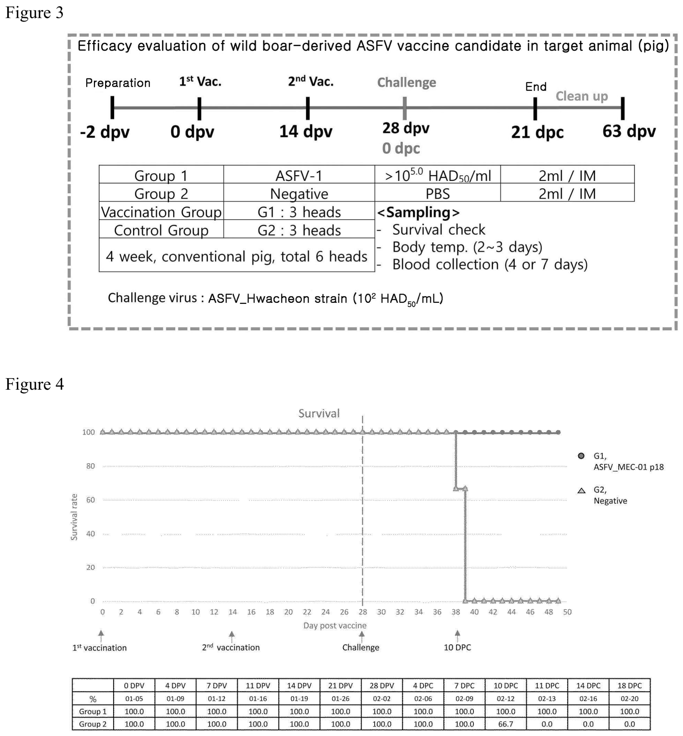

The inventors of the present invention conducted animal test to evaluate the attenuation (safety) and vaccine efficacy of the 18th-passaged attenuated ASF virus (ASFV-KOR.INJE.MEC-01.2022; ASFV_MEC-01), which was propagated and attenuated using the CA-CAS-01-A cell line. Briefly, 3- to 4-week-old pigs were intramuscularly injected with the vaccine candidate at a dose of >$10^5$ $HAD_{50}$/mL, administered twice in total at a 2-week interval. The pigs were then challenged with ASFV_Hwacheon strain ($10^2$ $HAD_{50}$/mL) (Figure 3). To monitor the detection level of virus, oral swabs, rectal swabs, nasal swabs, serum, and whole blood samples were collected every 2 to 3 days. Additionally, temperature changes, clinical symptoms, and survival rates were observed over a period of 3 weeks.

7. Statistical analysis

The Student's t-test was used for all statistical analyses, and a P-value of less than 0.05 was considered statistically significant.

Example 1. Characterization of Cell-Adapted ASFV Isolate

The ASFV strain isolated from wild boars in Korea (ASF/Korea/Wildbore/Inje-11893/2021) was infected into the CA-CAS-01-A cell line (deposit number: KCTC14568BP) and subjected to serial passage culture. The adaptation process of the ASFV isolate in cells was then analyzed using qPCR targeting the ASFV *P72* gene. As a result, as shown in Figure 1A, the Ct value began to drop below 20 after the 5th passage, and a Ct value of approximately 12 or lower was observed after the 16th passage. Additionally, the virus titer of the 18th-passage single vaccine candidate strain (ASFV_MEC-01), which was finally obtained through plaque assay in CA-CAS-01-A cells, is presented in Table 2 below.

[Table 2]

| Results of titer determination of vaccine candidate (ASFV-KOR.INJE.MEC-01.2022) | | | |
|---|---|---|---|
| | Titration (7dpi) | | |
| **ASFV Virus** | **CT-value (CA_CAS-01_A)** | **$HAD_{50}$/mL (Primary swine macrophage)** | **$TCID_{50}$/mL (CA_CAS-01_A)** |
| ASFV-KOR.INJE.MEC-01.2022 | 11.97 | 1.122 x $10^8$ | 3.981 x $10^5$ |

Additionally, the ASFV isolate (ASFV_MEC-01) passaged 18 times in total was infected into either the CA-CAS-01-A cell line or primary porcine macrophages, and its viral propagation over time was assessed using either $TCID_{50}$ (tissue culture infectious dose) or $HAD_{50}$ (median hemadsorption unit). The results indicated that even after adaptation passage in the CA-CAS-01-A cell line, the viral propagation capacity did not decrease compared to the original isolate - in fact, it showed a slight increase (Figures 1B and 1C).

Example 2. Genomic Analysis of Cell-Adapted ASFV Isolate

In order to determine the characteristics of the final secured vaccine candidate (ASFV_MEC-01), the virus was recovered from the culture broth after passaging for 18 times and its genome sequence was analyzed. As a result, it was found that a large gene deletion occurred in the 5' region, as illustrated in Figure 2. The genes located in the deleted region are summarized in Table 3, and, among the deleted genes, the genes associated with the attenuation are as described in Table 4. In addition, the positions of the deleted genes in Table 4 compared to the sequence of African swine fever virus isolate ASFV Georgia 2007/1 genome assembly, complete genome: monopartite (GenBank: FR682468.2) are as described in Table 5.

[Table 3]

**Genes in ASFV-KOR.INJE.MEC-01.2022 (ASFV_MEC-01) deletion site**

| Gene info | G_ACD and Other | ASFV_MEC-01 NGS | ASFV_MEC-01 PCR status |
|---|---|---|---|
| G ACD 00120 | G_ACD | Gene | Detected |
| G ACD 00160 | | Gene | Detected |
| G ACD 00190 | | Deletion | Not detected |
| G ACD 00210 | | | |
| G ACD 00240 | | | |
| G ACD 00270 | | | |
| G ACD00300 | | | |
| G ACD00320 | | | |
| G ACD00330 | | | |
| G ACD00350 | | Gene | Detected |
| G ACD00360 | | Gene | Not detected |
| G ACD00520 | | Deletion | Detected |
| X69R | Other | Gene | Detected |
| 285L | | Gene | Detected |
| I7L | | Gene | Detected |
| I8L | | Gene | Detected |
| I9R | | Gene | Detected |
| I10L | | Gene | Detected |
| L11L | | Gene | Detected |
| A224L | | Gene | Detected |
| EP153R | | Gene | Detected |
| EP402R | | Gene | Detected |
| NP868R | | Gene | Detected |
| P1192R | | Gene | Detected |
| E199L | | Gene | Detected |
| EP424R | | Gene | Detected |
| C962R | | Gene | Detected |
| NP419L | | Gene | Detected |
| I267L | | Gene | Detected |
| I329L | | Gene | Detected |
| I177L | | Gene | Detected |

| MGF | Gene info | ASFV_MEC-01 NGS | ASFV_MEC-01 PCR status |
|---|---|---|---|
| MGF 100 | 1L | Gene | Detected |
| | 1R | Deletion | Not detected |
| | 3L | Gene | Detected |
| MGF 110 | 1L | Gene | Detected |
| | 2L | Gene | Detected |
| | 3L | Gene | Detected |
| | 4L | Gene | Detected |
| | 5L-6L | Gene | Detected |
| | 7L | Gene | Detected |
| | 8L | Deletion (N terminal 80%) | Partial detected |
| | 9L | Deletion | Not detected |
| | 10L | Deletion | Not detected |
| | 14L | Deletion | Not detected |
| | 12L | Deletion | Not detected |
| | 13La | Deletion | Not detected |
| | 13Lb | Deletion | Not detected |
| MGF 300 | 1L | Deletion | Not detected |
| | 2R | Deletion | Not detected |
| | 4L | Deletion | Not detected |
| MGF 360 | 1La | Gene | Detected |
| | 1Lb | Gene | Detected |
| | 2L | Gene | Detected |
| | 3L | Gene | Detected |
| | 4L | Deletion | Not detected |
| | 6L | Deletion | Not detected |
| | 8L | Deletion (C terminal Frameshift 50%) | Partial detected |
| | 9L | Gene | Detected |
| | 10L | Gene | Detected |
| | 11L | Gene | Detected |
| | 12L | Gene | Detected |
| | 13L | Gene | Detected |
| | 14L | Gene | Detected |
| | 15R | Gene | Detected |
| | 16R | Gene | Detected |
| | 18R | Gene | Detected |
| | 19Ra | Gene | Detected |
| | 19Rb | Gene | Detected |
| | 21R | Gene | Detected |

[Table 4]

| Results of genomic analysis of attenuated ASFV_MEC-01 virus | |
|---|---|
| | Gene deleted |
| Deletion site | **MGF 100-1R, ASFV G ACD 00190, MGF 110-9L, MGF 110-10L, MGF 110-** |

(continued)

| Results of genomic analysis of attenuated ASFV_MEC-01 virus | |
|---|---|
| | Gene deleted |
| | **14L, ASFV G ACD 00240, MGF 110-12L, MGF 110-13La, MGF 110-13Lb, ASFV G ACD 00270, MGF 360-4L, MGF 360-6L, MGF 360-8L partial, MGF 300-1L, MGF 300-2R, MGF 300-4L,** *MGF 110-8L partial, ASFV G ACD 00210, ASFV G ACD 00300, ASFV G ACD 00320, ASFV G ACD 00330, ASFV G ACD 00350, ASFV G ACD 00360, X69R* |
| **Bold:** Genes affecting attenuation | |

[Table 5]

| Site of gene deletion | | | | |
|---|---|---|---|---|
| ASFV_MEC-01 | | | | Passage 18 |
| Deletion size | | | | 11,527 ~ 24,491 |
| Deletion gene | MGF 100 | 1R | 12056..12430 | Del |
| | MGF 110 | 8L | 11455..>11838 | Partial |
| | | 9L | 12590..13462 | Del |
| | | 10L - 14L fusion | 13753..14571 | Del |
| | | 12L | 14761..15120 | Del |
| | | 13La | 15206..15553 | Del |
| | | 13Lb | 15663..16031 | Del |
| | MGF 300 | 1L | 20704..21510 | Del |
| | | 2R | 22336..22818 | Del |
| | | 4L | 22908..23900 | Del |
| | MGF 360 | 4L | 16212..17375 | Del |
| | | 6L | 18189..19316 | Del |
| | | 8L | 24014..24973 | Partial |
| | G_ACD | G ACD 00190 | 12456..12581 | Del |
| | | G ACD 00210 | 13461..13652 | Del |
| | | G ACD 00240 | 14570..14680 | Del |
| | | G ACD 00270 | 16056..16169 | Del |
| | | G ACD 00300 | 17929..18045 | Del |
| | | G ACD 00320 | 19544..19669 | Del |
| | | G ACD 00330 | 19857..19970 | Del |
| | | G ACD 00350 | 19967..20098 | Del |
| | | G ACD 00360 | 20169..20285 | Del |
| | Other | X69R | 20197..20406 | Del |
| * Reference sequence : ASFV Georgia 2007/1 genome (GenBank : FR682468.2) | | | | |

Furthermore, in addition to the above gene deletions, many specific nucleotide mutations were identified in the genome sequence of the vaccine candidate ASFV_MEC-01 compared to the reference sequence (ASFV Georgia 2007/1 genome, GenBank: FR682468.2) and the genome sequence of the isolate (ASF/Korea/Wildbore/Inje-11893/2021).

[Table 6]

EP 4 722 352 A1

The vaccine candidate ASFV_MEC-01 of the present invention showed genetic stability (in terms of deleted site and location of point mutation) up to 20 passages.

### Results of genomic analysis of ASFV-KOR.INJE.MEC-01.2022 (ASFV_MEC-01) – Point mutation

| | Gene | MGF360-1La | MGF110-1L | MGF 360-10L | | MGF 505-9R | EP424R | | | MGF505-11L |
|---|---|---|---|---|---|---|---|---|---|---|
| | Function | Unknown | non-essential | Partial attenuated | | | mRNA modification | | | replication in macrophages |
| | Necleotide Position | 2,329 | 7,059 | 26,425 | 27,200 | 44,576 | 72,295 | 72,315 | 72,316 | 179,097 |
| | Amino acid change | L106P | W197* | N69S | N329S | K323E | silent mutation | | | M14I |
| Virus | Georgia/2007/1 | A | C | T | T | A | G | G | G | C |
| | ASF/Korea/Wildbore/Inje-11893/2021 | A | C | T | T | A | G | G | G | C |
| | ASFV-KOR.INJE.MEC-01.2022 | G | T | C | C | G | A | A | A | T |

| | Gene | MGF505-11L | MGF100-1L | | | NCR | MGF100-3L | I7L | | hypthetical |
|---|---|---|---|---|---|---|---|---|---|---|
| | Function | replication in macrophages | specific CD8+ T-cell epitopes | | | Unknown | Unknown | Partial virulence | | Unknown |
| | Necleotide Position | 180,319 | 180,607 | 180,619 | 180,718 | 181,034 | 181,322 | 181,809 | 181,891 | 182,053 |
| | Amino acid change | A422T | D96N | E100K | silent mutation | | A86T | silent mutation | | |
| Virus | Georgia/2007/1 | C | C | C | C | C | C | C | C | C |
| | ASF/Korea/Wildbore/Inje-11893/2021 | C | C | C | C | C | C | C | C | C |
| | ASFV-KOR.INJE.MEC-01.2022 | T | T | T | T | T | T | T | T | T |

Example 3. Safety and efficacy analysis of vaccine candidates

To evaluate the safety (attenuation) and efficacy of the final vaccine candidate ASFV_MEC-01, animal tests were conducted.

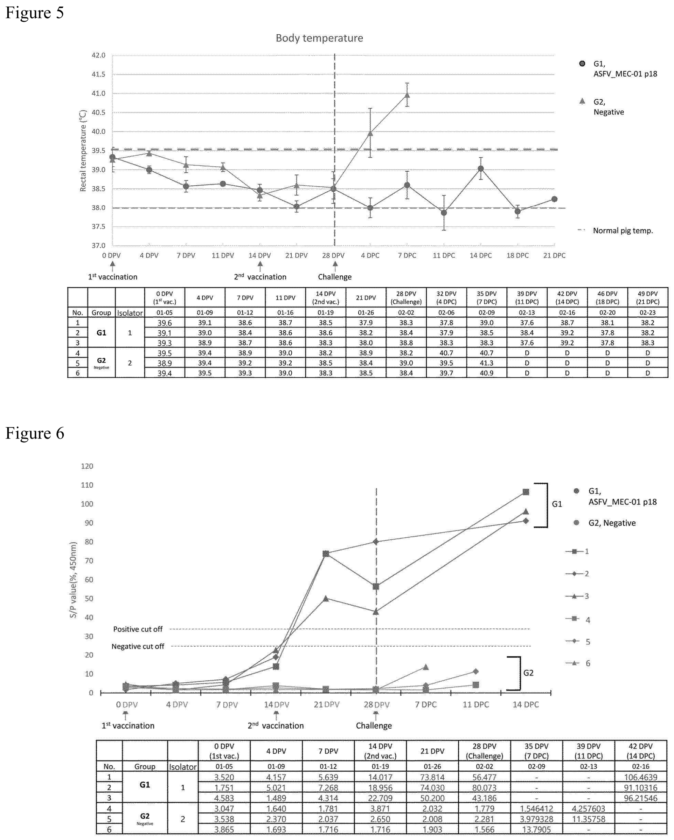

The vaccine candidate (ASFV_MEC-01) was administered twice at two-week intervals, followed by a challenge infection with ASFV_Hwacheon strain (isolated from wild boars in Korea). The three-week survival rate of the test animals was then assessed. The results showed that in the non-immunized group (i.e., not immunized with any vaccine candidate), mortality began on day 10 post-challenge, and all animals died by day 11. In contrast, the group immunized with the vaccine candidate demonstrated a 100% survival rate (Figure 4). Additionally, the group immunized with the vaccine candidate maintained normal body temperature throughout the vaccination period and post-challenge, showing no fever symptoms. However, in the non-vaccinated group, fever symptoms appeared from day 4 post-challenge (Figure 5). Furthermore, multi-antigen indirect ELISA analysis for ASFV p32, p62, and p72 proteins revealed that antibody titers gradually increased after the second vaccine candidate dose, turning seropositive within a week after the second dose of the vaccine. The antibody level remained high up to 14 days post-challenge (Figure 6). This strong antibody titer is believed to have contributed to protection against the ASFV_Hwacheon strain.

Based on the above results, it was found that, as the ASF vaccine candidate ASFV-KOR.INJE.MEC-01.2022 (ASFV_MEC-01) demonstrates both safety through natural attenuation and efficacy as a vaccine, it can be utilized as a valuable antigen for the development of an effective ASF vaccine.

## Claims

1. An attenuated African swine fever virus ASFV-KOR.INJE.MEC-01.2022 obtained by passaging African swine fever virus (ASFV) in a rhesus monkey kidney cell-derived cell line.

2. The attenuated African swine fever virus ASFV-KOR.INJE.MEC-01.2022 according to Claim 1, wherein the rhesus monkey kidney cell-derived cell line is CA-CAS-01-A cell line deposited with deposit number KCTC 14568BP.

3. The attenuated African swine fever virus ASFV-KOR.INJE.MEC-01.2022 according to Claim 1, wherein the attenuated African swine fever virus has deletion of one or more genes selected from the group consisting of *MGF 100-1R, ASFV G ACD 00190, MGF 110-9L, MGF 110-10L, MGF 110-14L, ASFV G ACD 00240, MGF 110-12L, MGF 110-13La, MGF 110-13Lb, ASFV G ACD 00270, MGF 360-4L, MGF 360-6L, MGF 360-8L partial, MGF 300-1L, MGF 300-2R, MGF 300-4L, MGF 110-8L partial, ASFV G ACD 00210, ASFV G ACD 00300, ASFV G ACD 00320, ASFV G ACD 00330, ASFV G ACD 00350, ASFV G ACD 00360* and *X69R*.

4. The attenuated African swine fever virus ASFV-KOR.INJE.MEC-01.2022 according to Claim 3, wherein the attenuated African swine fever virus has deletion of *MGF 100-1R, ASFV G ACD 00190, MGF 110-9L, MGF 110-10L, MGF 110-14L, ASFV G ACD 00240, MGF 110-12L, MGF 110-13La, MGF 110-13Lb, ASFV G ACD 00270, MGF 360-4L, MGF 360-6L, MGF 360-8L partial, MGF 300-1L, MGF 300-2R, MGF 300-4L, MGF 110-8L partial, ASFV G ACD 00210, ASFV G ACD 00300, ASFV G ACD 00320, ASFV G ACD 00330, ASFV G ACD 00350, ASFV G ACD 00360* and *X69R* genes.

5. The attenuated African swine fever virus ASFV-KOR.INJE.MEC-01.2022 according to Claim 4, wherein the attenuated African swine fever virus additionally comprises any of the following mutations: based on ASFV Georgia 2007/1 genome sequence, the $2{,}329^{th}$ base adenine (A) is changed to guanine (G), the $7{,}059^{th}$ base cytosine (C) is changed to thymine (T); the $26{,}425^{th}$ and $27{,}200^{th}$ base thymine (T) is changed to cytosine (C), the $44{,}576^{th}$ base adenine (A) is changed to guanine (G); the $72{,}295^{th}$, $72{,}315^{th}$, and $72{,}316^{th}$ base guanine (G) is changed to adenine (A), and cytosine (C), which is the $179{,}097^{th}$, $180{,}319^{th}$, $180{,}607^{th}$, $180{,}619^{th}$, $180{,}718^{th}$, $181{,}034^{th}$, $181{,}322^{nd}$, $181{,}809^{th}$, $181{,}891^{st}$ and $182{,}053^{rd}$ base, is changed to thymine (T).

6. A vaccine composition for prevention of African swine fever, comprising the attenuated African swine fever virus ASFV-KOR.INJE.MEC-01.2022 of any one of Claims 1 to 5 as an effective component.

7. The vaccine composition according to Claim 6, wherein the vaccine composition additionally comprises a pharmaceutically acceptable carrier or adjuvant.

8. A method for preventing African swine fever by administering the vaccine composition of Claim 6 to an animal of family *Suidae*.

9. A kit for detecting African swine fever virus, which comprises the attenuated African swine fever virus ASFV-KOR.INJE.MEC-01.2022 of any one of Claims 1 to 5 as an effective component.

10. A method for diagnosing African swine fever infection comprising reacting the attenuated African swine fever virus ASFV-KOR.INJE.MEC-01.2022 of any one of Claims 1 to 5 with antiserum isolated from a suspected African swine fever-infected individual.

11. A method for producing a vaccine against African swine fever comprising passaging the attenuated African swine fever virus ASFV-KOR.INJE.MEC-01.2022 of any one of Claims 1 to 5 in a vaccine-producing cell line.

Figure 1

Figure 2

ASFV_MEC-01 Deletion site analysis

Figure 3

Efficacy evaluation of wild boar−derived ASFV vaccine candidate in target animal (pig)

Preparation    **1st Vac.**    **2nd Vac.**    Challenge    End   Clean up

**-2 dpv**    **0 dpv**    **14 dpv**    **28 dpv**    **21 dpc**    **63 dpv**

**0 dpc**

| Group 1 | ASFV-1 | $>10^{5.0}$ HAD$_{50}$/ml | 2ml / IM |
|---|---|---|---|
| Group 2 | Negative | PBS | 2ml / IM |
| Vaccination Group | G1 : 3 heads | <Sampling> | |
| Control Group | G2 : 3 heads | - Survival check | |
| 4 week, conventional pig, total 6 heads | | - Body temp. (2~3 days) | |
| | | - Blood collection (4 or 7 days) | |

Challenge virus : ASFV_Hwacheon strain ($10^2$ HAD$_{50}$/mL)

Figure 4

Survival

G1, ASFV_MEC-01 p18

G2, Negative

1st vaccination    2nd vaccination    Challenge    10 DPC

| % | 0 DPV | 4 DPV | 7 DPV | 11 DPV | 14 DPV | 21 DPV | 28 DPV | 4 DPC | 7 DPC | 10 DPC | 11 DPC | 14 DPC | 18 DPC |
|---|---|---|---|---|---|---|---|---|---|---|---|---|---|
| | 01-05 | 01-09 | 01-12 | 01-16 | 01-19 | 01-26 | 02-02 | 02-06 | 02-09 | 02-12 | 02-13 | 02-16 | 02-20 |
| Group 1 | 100.0 | 100.0 | 100.0 | 100.0 | 100.0 | 100.0 | 100.0 | 100.0 | 100.0 | 100.0 | 100.0 | 100.0 | 100.0 |
| Group 2 | 100.0 | 100.0 | 100.0 | 100.0 | 100.0 | 100.0 | 100.0 | 100.0 | 100.0 | 66.7 | 0.0 | 0.0 | 0.0 |

Figure 5

Body temperature

| No. | Group | Isolator | 0 DPV (1st vac.) 01-05 | 4 DPV 01-09 | 7 DPV 01-12 | 11 DPV 01-16 | 14 DPV (2nd vac.) 01-19 | 21 DPV 01-26 | 28 DPV (Challenge) 02-02 | 32 DPV (4 DPC) 02-06 | 35 DPV (7 DPC) 02-09 | 39 DPV (11 DPC) 02-13 | 42 DPV (14 DPC) 02-16 | 46 DPV (18 DPC) 02-20 | 49 DPV (21 DPC) 02-23 |
|---|---|---|---|---|---|---|---|---|---|---|---|---|---|---|---|
| 1 | G1 | 1 | 39.6 | 39.1 | 38.6 | 38.7 | 38.5 | 37.9 | 38.3 | 37.8 | 39.0 | 37.6 | 38.7 | 38.1 | 38.2 |
| 2 | | | 39.1 | 39.0 | 38.4 | 38.6 | 38.6 | 38.2 | 38.4 | 37.9 | 38.5 | 38.4 | 39.2 | 37.8 | 38.2 |
| 3 | | | 39.3 | 38.9 | 38.7 | 38.6 | 38.6 | 38.0 | 38.8 | 38.3 | 38.3 | 37.6 | 39.2 | 37.8 | 38.3 |
| 4 | G2 Negative | 2 | 39.5 | 39.4 | 38.9 | 39.0 | 38.2 | 38.9 | 38.2 | 40.7 | 40.7 | D | D | D | D |
| 5 | | | 38.9 | 39.4 | 39.2 | 39.2 | 38.5 | 38.4 | 39.0 | 39.5 | 41.3 | D | D | D | D |
| 6 | | | 39.4 | 39.5 | 39.3 | 39.0 | 38.3 | 38.5 | 38.4 | 39.7 | 40.9 | D | D | D | D |

Figure 6

| No. | Group | Isolator | 0 DPV (1st vac.) 01-05 | 4 DPV 01-09 | 7 DPV 01-12 | 14 DPV (2nd vac.) 01-19 | 21 DPV 01-26 | 28 DPV (Challenge) 02-02 | 35 DPV (7 DPC) 02-09 | 39 DPV (11 DPC) 02-13 | 42 DPV (14 DPC) 02-16 |
|---|---|---|---|---|---|---|---|---|---|---|---|
| 1 | G1 | 1 | 3.520 | 4.157 | 5.639 | 14.017 | 73.814 | 56.477 | - | - | 106.4639 |
| 2 | | | 1.751 | 5.021 | 7.268 | 18.956 | 74.030 | 80.073 | - | - | 91.10316 |
| 3 | | | 4.583 | 1.489 | 4.314 | 22.709 | 50.200 | 43.186 | - | - | 96.21546 |
| 4 | G2 Negative | 2 | 3.047 | 1.640 | 1.781 | 3.871 | 2.032 | 1.779 | 1.546412 | 4.257603 | - |
| 5 | | | 3.538 | 2.370 | 2.037 | 2.650 | 2.008 | 2.281 | 3.979328 | 11.35758 | - |
| 6 | | | 3.865 | 1.693 | 1.716 | 1.716 | 1.903 | 1.566 | 13.7905 | - | - |

# INTERNATIONAL SEARCH REPORT

| International application No. |
| --- |
| **PCT/KR2024/007169** |

| A. | CLASSIFICATION OF SUBJECT MATTER |
| --- | --- |

**C12N 7/00**(2006.01)i; **A61K 35/76**(2006.01)i; **A61P 31/20**(2006.01)i; **G01N 33/569**(2006.01)i

According to International Patent Classification (IPC) or to both national classification and IPC

| B. | FIELDS SEARCHED |
| --- | --- |

Minimum documentation searched (classification system followed by classification symbols)

C12N 7/00(2006.01); A61K 39/12(2006.01); A61P 31/12(2006.01); A61P 31/20(2006.01); G01N 33/80(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아프리카돼지열병 바이러스(african swine fever virus), 레수스 원숭이 신장 세포 (rhesus monkey kidney cell), 계대배양(subculture), 백신(vaccine), ASFV-KOR.INJE.MEC-01.2022

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y<br>A | MELONI, D. et al. Cell Lines for the Development of African Swine Fever Virus Vaccine Candidates: An Update. Vaccines. 2022, vol. 10, document 707, pp. 1-25.<br>See abstract; pages 5-10; and table 1. | 1,6-11<br>2-5 |
| Y | WO 2022-140364 A2 (VST LLC DBA MEDGENE LABS) 30 June 2022 (2022-06-30)<br>See abstract; and paragraphs [0013], [00128], [00129] and [00269]. | 1,6-11 |
| A | TABARES, E. et al. African swine fever virus DNA: deletions and additions during adaptation to growth in monkey kidney cells. Archives of Virology. 1987, vol. 97, pp. 333-346.<br>See entire document. | 1-11 |
| A | US 2021-0333293 A1 (THE UNITED STATES OF AMERICA, AS REPRESENTED BY THE SECRETARY OF AGRICULTURE) 28 October 2021 (2021-10-28)<br>See entire document. | 1-11 |

☑ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **19 September 2024** | **19 September 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

# EP 4 722 352 A1

**INTERNATIONAL SEARCH REPORT**

| International application No. |
| --- |
| **PCT/KR2024/007169** |

## C. DOCUMENTS CONSIDERED TO BE RELEVANT

| Category* | Citation of document, with indication, where appropriate, of the relevant passages | Relevant to claim No. |
| --- | --- | --- |
| A | EP 4043030 A1 (NATIONAL AGRICULTURE AND FOOD RESEARCH ORGANIZATION) 17 August 2022 (2022-08-17)<br><br>See entire document. | 1-11 |

Form PCT/ISA/210 (second sheet) (July 2022)

18

**INTERNATIONAL SEARCH REPORT**

International application No.

**PCT/KR2024/007169**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2024/007169**

| Patent document cited in search report | | | Publication date (day/month/year) | Patent family member(s) | | | Publication date (day/month/year) |
|---|---|---|---|---|---|---|---|
| WO | 2022-140364 | A2 | 30 June 2022 | AU | 2021-409533 | A1 | 30 June 2022 |
| | | | | CA | 3202937 | A1 | 30 June 2022 |
| | | | | CN | 116802307 | A | 22 September 2023 |
| | | | | EP | 4262865 | A2 | 25 October 2023 |
| | | | | JP | 2024-506747 | A | 14 February 2024 |
| | | | | KR | 10-2023-0135072 | A | 22 September 2023 |
| | | | | MX | 2023007395 | A | 15 September 2023 |
| | | | | US | 11844831 | B2 | 19 December 2023 |
| | | | | US | 2022-0193218 | A1 | 23 June 2022 |
| | | | | WO | 2022-140364 | A3 | 18 August 2022 |
| US | 2021-0333293 | A1 | 28 October 2021 | US | 11796548 | B2 | 24 October 2023 |
| | | | | WO | 2021-216825 | A1 | 28 October 2021 |
| EP | 4043030 | A1 | 17 August 2022 | JP | 2021-061772 | A | 22 April 2021 |
| | | | | JP | 7382628 | B2 | 17 November 2023 |
| | | | | US | 2024-0068052 | A1 | 29 February 2024 |
| | | | | WO | 2021-070407 | A1 | 15 April 2021 |

Form PCT/ISA/210 (patent family annex) (July 2022)

## REFERENCES CITED IN THE DESCRIPTION

**Patent documents cited in the description**

- KR 20210065128
- KR 20210127887

- FR 682468

**Non-patent literature cited in the description**

- **DIXON et al.** *Virus Res*, 2013, vol. 173, 3-14